# EUROPEAN PATENT APPLICATION

(11) **EP 1 417 950 A1**
(43) Date of publication of application: **12.05.2004**
(21) Application number: 03078477.1
(22) Date of filing: 05.11.2003
(51) Int. Cl.: A61J 1/00, A61M 5/14

(54) **Device for the administration of nutritional mixtures by parenteral route**

(30) Priority: 08.11.2002 IT MI20022380
(71) Applicant: HAEMOPHARM INDUSTRY AG, 9490 Vaduz (LI)
(72) Inventor: Valoti, Michele, 6900 Massagno (CH)
(74) Representative: Mittler, Enrico

(57) **Abstract**

A device for the administration of nutritional mixtures by parenteral route, comprises a bag container (1) for nutritional mixture provided with an inflow duct (3) with a closing clip (7) and with an outflow duct (4) provided with a stopper (13), a duct (9) with multiple inlets for the connection of said inflow duct (3) with a plurality of bags or bottles (10) for the containment of mixtureable liquid substances and an infusion set (20) for the parenteral delivery of said nutritional mixture to a patient. The infusion set (20) is permanently connected with the outflow duct (4) of the bag container (1) and its stopper (13) is of a type which is frangible by bending said outflow duct (4).

## Description

The present invention concerns a device for the administration of nutritional mixtures by parenteral route.

When a patient is not capable to take food in normal way, one resorts to parenteral administration of nutritional mixtures, that are prepared in liquid form inside of a bag, from which they are then taken and progressively introduces into a vein of the patient through an infusion set with outflow duct provided with a needle for intravene.

Normally the bag provides an outflow duct that is separate from the infusion set and closed by a membrane, that at the moment of its use gets perforated by a needle located on the top of the infusion set, that works as an inlet for the nutritional mixture.

In such known devices the execution of the perforation can be source of contamination and/or to determine a spill of the product.

Scope of the present invention is to provide a device for the administration of nutritional mixtures by parenteral route that prevents the inconveniences above described.

According to the present invention such scope is attained with a device comprising a bag container for nutritional mixture provided with an inflow duct with a closing clip and with an outflow duct provided with a stopper, a multiple inflow duct for the connection of said inflow duct with a plurality of bags or bottles for the containment of mixtureable liquid substances and an infusion set for the parenteral delivery of said nutritional mixture to a patient, characterised in that said infusion set is permanently connected with said outflow duct of the bag container and said stopper is of a type which is frangible through bending of said outflow duct.

It is evident that in this way the nutritional mixture can never get in contact with the external atmosphere and any danger of its waste and/or contamination is thus prevented. In short the device according to the invention creates a sealed circuit that is inaccessible from the outside up to the moment of the delivery of the nutritional mixture to the patient.

The characteristics and the advantages of the present invention will become evident from the following detailed description of an embodiment thereof, that is illustrated as a non limiting example in the enclosed drawings, in which:
Figure 1 is a schematic representation of a device for the administration of nutritional mixtures according to the present invention , as viewed during the stage of introduction of the mixtureture into the bag container;
Figure 2 is a schematic representation similar to the one in Figure 1 with the bag container in the stage of delivery of the mixtureture;
Figure 3 shows a magnified sectional view of the outflow duct of the bag container when it is closed by a frangible stopper;
Figure 4 is a sectional view similar to the one in Figure 3 with the frangible stopper broken for the opening of said outflow duct.

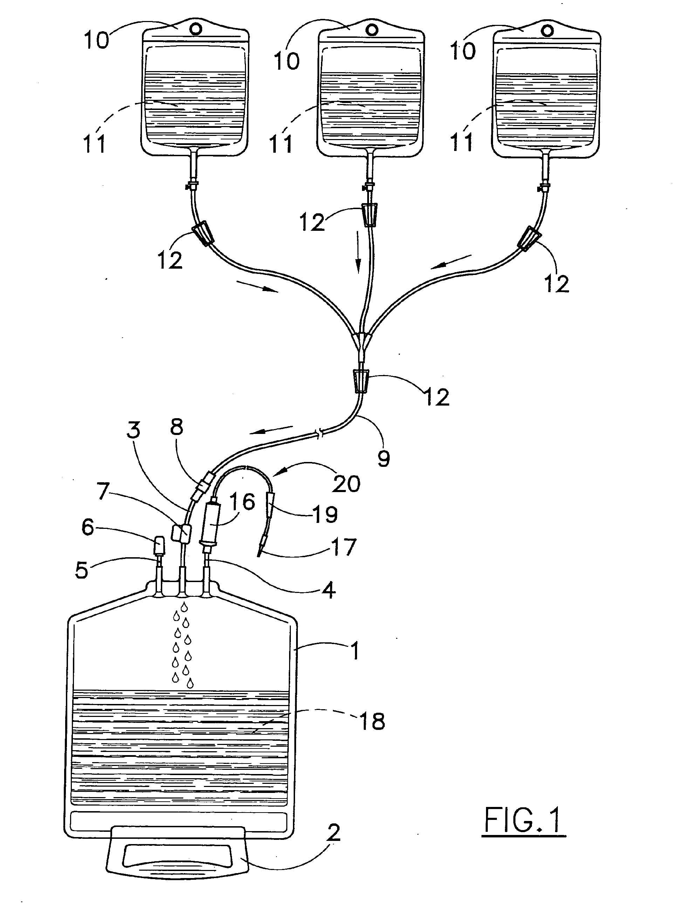

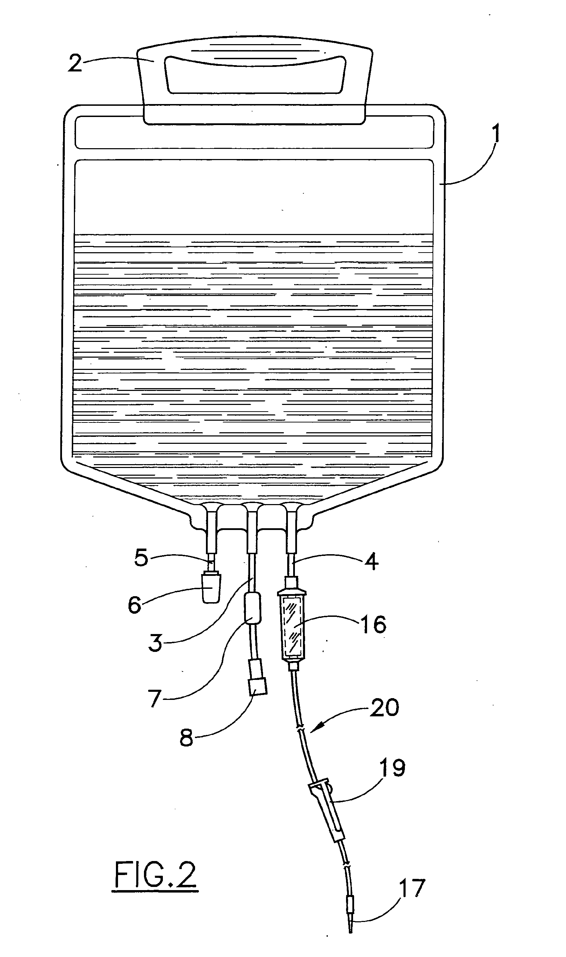

The device in Figures 1 and 2 comprises a flexible bag container 1 provided with a holding handle 2, with an inflow duct 3, an outflow duct 4 and an emptying duct 5, the latter provided with permanent closing stopper 6.

The inflow duct 3 is provided with a closing clip 7 normally open and with a terminal screw connection 8 for its separable connection with a flexible duct 9 with three inlets which lead to respective bags or bottles 10 containing liquid substances 11 mixtureable to each other in order to form the desired nutritional mixture. The flexible duct 9 is provided with closing clips 12 that are normally open.

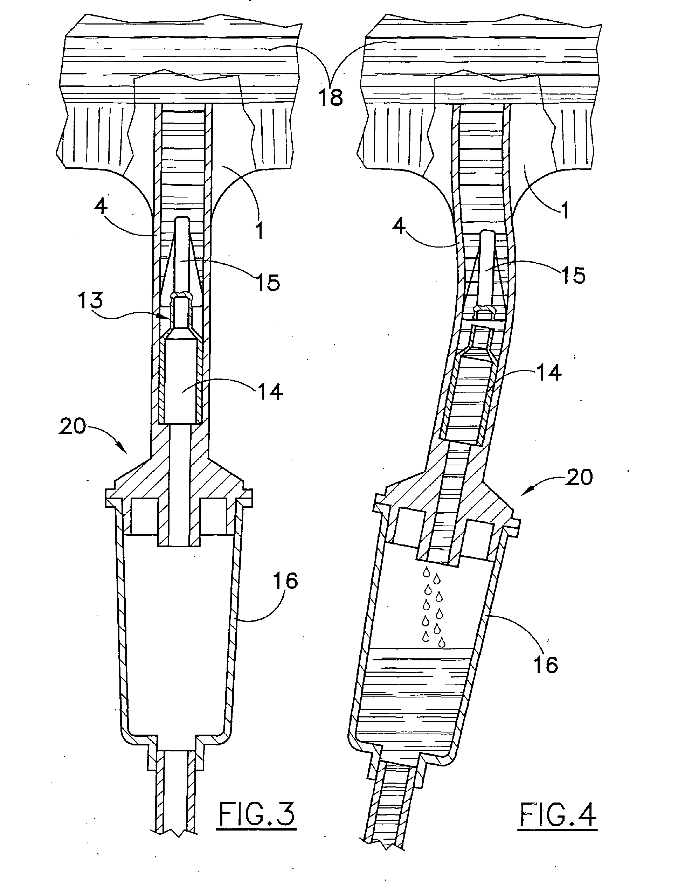

The outflow duct 4, as shown in detail in Figure 3, is provided with a frangible closing stopper 13 made up of a hollow cylindrical part 14 and of a finned part 15 placed for the closing of the internal passage of said hollow part and has permanently attached to the same duct an infusion set 20 made up of a small tank 16 followed by a flow regulator 19 and possibly ending with a needle 17 for its connection with the vein of a patient.

In the stage of introduction of the nutritional mixture into the container 1 the latter is turned upside-down as in Figure 1 and the clips 12 get opened in order to allow the liquid substances 11 to flow into the container 1 in order to form a nutritional mixture 18.

Once the filling is over the clip 7 gets closed in permanent and inviolable way, the screw connection 8 gets open in order to allow the separation of the duct 9 from the container 1 and the latter is finally positioned and hung in the condition of Figure 2.

For the delivery of the nutritional mixture 18 to a patient, with the flow regulator 19 closed, the frangible stopper 13 gets broken by bending the outflow duct as shown in Figure 4. The finned part 15 therefore separates from the hollow part 14, thus opening the communication between the container 1 and the infusion set 20.

At this point, by following the normal hypodermoclysis procedures, a transversal pressure is exerted onto the small tank 16 which has the scope to create an air trap inside the same small tank. The nutritional mixture partially fills the small tank 16 and then the flow regulator 19 gets opened in order to make the liquid above flow down to the needle 17 in order to completely eliminate possible air present in the infusion set. Once the flow regulator 19 gets closed, the needle 17 or other possible needle provided at the moment is inserted into the vein of the patient and the flow regulator 19 opens again.

The nutritional mixture therefore outflows drop by drop into the vein of the patient.

Since there is never any connection between the outflow duct 4 and the external atmosphere, any spill of product and/or its contamination is prevented.

## Claims

1. Device for the administration of nutritional mixtures by parenteral route, comprising a bag container (1) for nutritional mixture provided with an inflow duct (3) with a closing clip (7) and with an outflow duct (4) provided with a stopper (13), a duct (9) with multiple inlets for the connection of said inflow duct (3) with a plurality of bags or bottles (10) for the containment of mixtureable liquid substances and an infusion set (20) for the parenteral delivery of said nutritional mixture to a patient, **characterised in that** said infusion set (20) is permanently connected with said outflow duct (4) of the bag container (1) and said stopper (13) is of a type which is frangible by bending said outflow duct (4).

2. Device according to claim 1, **characterised in that** said frangible stopper (13) is made up of a hollow cylindrical part (14) and of a finned part (15) placed for the closing of said hollow part (14) and separable from it by bending said outflow duct (4).
